# EUROPEAN PATENT APPLICATION

(11) **EP 3 970 795 A1**
(43) Date of publication of application: **23.03.2022**
(21) Application number: 20804842.1
(22) Date of filing: 14.05.2020
(51) Int. Cl.: A61P 27/02, C12N 5/07, C12N 5/079, A61K 35/30

(54) **METHOD FOR PURIFYING NEURAL CREST CELLS OR CORNEAL EPITHELIAL CELLS**

(30) Priority: 15.05.2019 JP 2019091988; 09.10.2019 JP 2019186280
(71) Applicant: AJINOMOTO CO., INC., Chuo-ku Tokyo 104-8315 (JP)
(72) Inventor: BABA, Shizuka, Kawasaki-shi, Kanagawa 210-8681 (JP); TAKAHASHI, Kazuma, Kawasaki-shi, Kanagawa 210-8681 (JP); KONISHI, Atsushi, Kawasaki-shi, Kanagawa 210-8681 (JP)
(74) Representative: Hoffmann Eitle
(86) International application number: PCT/JP2020/019173
(87) International publication number: WO 2020/230832

(57) **Abstract**

A method for purifying neural crest cells or corneal epithelial cells is provided by the present invention.

## Description

### [Technical Field]

The present invention relates to a purification method of neural crest cells, and the like. In detail, it relates to a purification method of neural crest cells by using laminin 211, and the like. In other embodiment, the present invention relates to a purification method of corneal epithelial cells by using laminin 332, and the like.

### [Background Art]

In recent years, the development of novel treatment methods for diseases associated with cell transplantation is making great progress with the establishment of iPS cells. As one of the cells that can be suitably used for cell transplantation treatment, neural crest cell, which has a wide range of multipotency and is therefore also called "the 4th germ layer" is attracting attention.

Plural methods for preparing neural crest cells have been reported. For example, non-patent document 1 discloses method in which differentiation of iPS cell is induced in the presence of a TGF-β signal inhibitor, a Wnt signal activator, and the like, a cell population containing neural crest cells is prepared, only neural crest cells are collected from the cell population by using a cell sorter, and the cells are expansion cultured. Also, non-patent document 2 discloses that a neural crest cell with a comparatively high purity can be prepared by inducing differentiation of ES cells in the presence of FGF2 and TGFβ signal inhibitors, and the like, preparing a cell population containing neural crest cells, and further culturing the cell population in the presence of FGF2 and TGF-β inhibitors on a gelatin-containing medium for a long term.

In the field of cell transplantation treatment, corneal epithelial cell is also one of the cells that are attracting attention. The corneal epithelial cell constitutes the surface of the cornea, and acts as a barrier to protect the cornea from the outside world and has the ability to bring oxygen into the cornea. Many of the disorders of corneal epithelial cells lead to poor vision and significantly reduce the patient's quality of life. Therefore, there is a high great need to establish a method for efficiently producing transplantable corneal epithelial cells for treating such disorders.

As a method for preparing corneal epithelial cells, for example, a method for inducing the differentiation of corneal epithelial cells by culturing pluripotent stem cells in a serum-free medium containing no BMP for a certain period of time in the presence of stromal cells or amniotic membrane-derived factors has been reported (patent document 1).

### [Document List]

### [Patent document]

patent document 1: JP-B- 5759536

### [Non-patent documents]

non-patent document 1: Fukuta M. et al., PLoS One. 2014 Dec 2; 9(12):e112291.
non-patent document 2: Serrano F. et al., Stem Cells Dev. 2019 Jan 15; 28(2):81-100.

### [Summary of Invention]

### [Technical Problem]

Neural crest cells can be prepared using the methods disclosed in non-patent documents 1 and 2. However, these methods still embrace problems. For example, in the method disclosed in non-patent document 1, the process of collecting neural crest cells by a cell sorter is essential. However, the use of a cell sorter has various problems such as the problem of yield, purity, survival rate, and functional deterioration of the sorting-target cells, the cost of purchasing and maintaining the cell sorter, and the like. In addition, even though the method of non-patent document 2 does not involve the use of a cell sorter, it requires continuous passage to obtain high-purity neural crest cells, and its preparation takes a relatively long period of time, which poses a problem in efficiency.

Also, corneal epithelial cells can be prepared using the method disclosed in patent document 1. Similar to the above-mentioned neural crest cells, however, a step of selecting corneal epithelial cells from a cell population containing induced corneal epithelial cells by using a cell sorter and the like is required to obtain high-purity corneal epithelial cells by using the method, and the method has a problem in the efficiency.

### [Solution to Problem]

The present inventors have conducted intensive studies of the above-mentioned problems and found that neural crest cells can be purified by expansion culturing a cell population containing neural crest cells with laminin 211 as a scaffold, even without sorting neural crest cells by a cell sorter or the like. The present inventors have also found that neural crest cells can be purified in an extremely shorter period of time compared to conventional purification methods by using this method. The present inventors have further found that corneal epithelial cells can be purified by expansion culturing a cell population containing more than a given proportion of corneal epithelial cells by using laminin 332 as a scaffold, even without sorting corneal epithelial cells by a cell sorter or the like. Based on such findings, they have conducted further studies and completed the present invention. Accordingly, the present invention provides the following.

[1] A method for purifying a neural crest cell, comprising the following steps:
   step 1) obtaining a cell population comprising neural crest cells, and
   step 2) expansion culturing the cell population obtained in step 1, by using laminin 211 as a scaffold.
[2] The method of [1], wherein the cell population comprising neural crest cells obtained in step 1 is subjected to step 2 without being subjected to a sorting treatment of the neural crest cells.
[3] The method of [1] or [2], wherein the culture period in step 2 is 1 - 21 days.
[4] The method of any of [1] to [3], wherein the cell population comprising neural crest cells is derived from a pluripotent stem cell.
[5] The method of [4], wherein the pluripotent stem cell is an iPS cell.
[6] A method for producing a purified neural crest cell, comprising the following steps:
   step 1) obtaining a cell population comprising neural crest cells, and
   step 2) expansion culturing the cell population obtained in step 1, by using laminin 211 as a scaffold.
[7] The method of [6], wherein the cell population comprising neural crest cells obtained in step 1 is subjected to step 2 without being subjected to a sorting treatment of the neural crest cells.
[8] The method of [6] or [7], wherein the culture period in step 2 is 1 - 21 days.
[9] The method of any of [6] to [8], wherein the cell population comprising neural crest cells is derived from a pluripotent stem cell.
[10] The method of [9], wherein the pluripotent stem cell is an iPS cell.
[11] A method for purifying a corneal epithelial cell, comprising the following steps:
   step 1) obtaining a cell population comprising corneal epithelial cells in a proportion of not less than 25%, and
   step 2) expansion culturing the cell population obtained in step 1, by using laminin 332 as a scaffold.
[12] The method of [11], wherein the cell population comprising corneal epithelial cells obtained in step 1 is subjected to step 2 without being subjected to a sorting treatment of the corneal epithelial cells.
[13] The method of [11] or [12], wherein the laminin 332 used as the scaffold in the expansion culture in step 2 is coated in an amount of more than 0.01 µg/cm² and less than 0.5 µg/cm².
[14] The method of any of [11] to [13], wherein the cell population comprising corneal epithelial cells is derived from a pluripotent stem cell.
[15] The method of [14], wherein the pluripotent stem cell is an iPS cell.
[16] A method for producing a purified corneal epithelial cell, comprising the following steps:
   step 1) obtaining a cell population comprising corneal epithelial cells in a proportion of not less than 25%, and
   step 2) expansion culturing the cell population obtained in step 1, by using laminin 332 as a scaffold.
[17] The method of [16], wherein the cell population comprising corneal epithelial cells obtained in step 1 is subjected to step 2 without being subjected to a sorting treatment of the corneal epithelial cells.
[18] The method of [16] or [17], wherein the laminin 332 used as the scaffold in the expansion culture in step 2 is coated in an amount of more than 0.01 µg/cm² and less than 0.5 µg/cm².
[19] The method of any of [16] to [18], wherein the cell population comprising corneal epithelial cells is derived from a pluripotent stem cell.
[20] The method of [19], wherein the pluripotent stem cell is an iPS cell.

### [Advantageous Effects of Invention]

According to the present invention, neural crest cells can be purified highly conveniently in a short period of time from a cell population containing neural crest cells. According to the present invention, moreover, high-purity neural crest cells can be prepared highly conveniently in a short period of time.

Furthermore, according to the present invention, corneal epithelial cells can be purified highly conveniently from a cell population containing corneal epithelial cells. According to the present invention, moreover, high-purity corneal epithelial cells can be prepared highly conveniently.

### [Description of Embodiments]

The present invention is explained in detail in the following.

### 1. Purification method of neural crest cells

The present invention provides a method for purifying a neural crest cell, including the following steps (hereinafter sometimes to be referred to as "purification method 1 of the present invention"):
step 1) preparing a cell population containing neural crest cells, and step 2) expansion culturing the cell population obtained in step 1, by using laminin 211 as a scaffold.

"Neural Crest Cell (also referred to as "NCC")" means a cell that de-epithelializes from the structure of the neural crest that is temporarily formed between the epidermal ectoderm and the neural plate during the early development of vertebrates and is induced into various parts in the embryo body after transition from epithelium to mesenchyme. The term "neural crest cell" in the present specification includes not only the cells collected from living organisms but also pluripotent stem cell-derived neural crest cells, and cells that have been passaged therefrom. In the purification method of the present invention, the origin of the neural crest cells is not particularly limited, and they may be derived from any vertebrate, and neural crest cells derived from a mammal are preferred. Examples of such mammal include, but are not limited to, mouse, rat, guinea pig, hamster, rabbit, cat, dog, sheep, swine, bovine, horse, goat, monkey, and human.

As a method for preparing a cell population containing neural crest cells in step 1 of the purification method 1 of the present invention, when the cell population contains neural crest cells derived from living organisms, it can be prepared by recovering a cell population from living organism neural crest-derived tissue (e.g., bone marrow, spinal cord dorsal root ganglion, heart, cornea, iris, pulp, olfactory mucosa, etc.). In the case of a cell population containing neural crest cells derived from pluripotent stem cell, as described above, plural preparation methods are known. One example is a method of culturing pluripotent stem cells in a culture medium containing a TGFβ inhibitor and a GSK-3β inhibitor. By inducing differentiation of iPS cells in a culture medium containing a TGFβ inhibitor and a GSK-3β inhibitor, the iPS cells are differentiated into a self-formed ectodermal autonomous multi-zone (SEAM) constituted of various cell lineages of the eye. SEAM may contain neural crest cells.

Whether the thus-obtained cell population contains neural crest cells may be known by confirming the expression of one or more neural crest cell-specific marker genes such as TFAP2a, SOX9, SOX10, TWISTI, PAX3 and the like by a method known per se. In addition, the proteins present on the cellular surface of neural crest cells, such as CD271 protein (also referred to as "p75(NTR)") and the like, can also be used as neural crest cell-specific markers. It is preferable that the cell population containing neural crest cells used in the purification method 1 of the present invention is derived from pluripotent stem cell, more preferably, iPS cell.

In the present invention, the pluripotent stem cell is a stem cell having pluripotency that permits differentiation into many cells existing in the living body and also having proliferation potency. It encompasses any cells that are induced into intermediate mesodermal cells used in the present invention. Without particular limitation, pluripotent stem cells include, for example, embryonic stem (ES) cells, clone embryo-derived embryonic stem (ntES) cells obtained by nuclear transplantation, sperm stem cells (GS cells), embryonic germ cells (EG cells), induced pluripotent stem (iPS) cells, pluripotent cells derived from cultured fibroblasts or myeloid stem cells (Muse cells), and the like. Preferred pluripotent stem cells are iPS cells, more preferably human iPS cells, from the aspect that they can be obtained without destroying embryo, ovum and the like in the production step.

Production methods of iPS cells are known in the art and the cells can be produced by introducing a reprogramming factor into any somatic cell. Here, examples of the reprogramming factor include genes and gene products such as Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tel1, beta-catenin, Lin28b, Sall1, Sall4, Esrrb, Nr5a2, Tbx3, Glis1 and the like. These reprogramming factors may be used alone or in combination. Combination of reprogramming factors includes, for example, the combinations described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, wo2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26:2467-2474, Huangfu D, et al. (2008), Nat. Biotechnol. 26:1269-1275, Shi Y, et al. (2008), Cell Stem Cell, 3, 568-574, Zhao Y, et al. (2008), Cell Stem Cell, 3:475-479, Marson A, (2008), Cell Stem Cell, 3, 132-135, Feng B, et al. (2009), Nat. Cell Biol. 11:197-203, R.L. Judson et al., (2009), Nat. Biotechnol., 27:459-461, Lyssiotis CA, et al. (2009), Proc Natl Acad Sci U S A. 106:8912-8917, Kim JB, et al. (2009), Nature. 461:649-643, Ichida JK, et al. (2009), Cell Stem Cell. 5:491-503, Heng JC, et al. (2010), Cell Stem Cell. 6:167-74, Han J, et al. (2010), Nature. 463:1096-100, Mali P, et al. (2010), Stem Cells. 28:713-720, and Maekawa M, et al. (2011), Nature. 474:225-9.

Somatic cells include, nonrestrictively, fetal somatic cells, neonatal somatic cells, and mature healthy or diseased somatic cells, and also include primary cultured cells, subcultured cells, and established lines of cells encompassed in. Specifically, somatic cell is exemplified by (1) tissue stem cells (somatic stem cells) such as neural stem cells, hematopoietic stem cells, mesenchymal stem cells, pulp stem cells, and the like, (2) tissue progenitor cells, (3) differentiated cells such as blood cells (peripheral blood cells, cord blood cells, etc.), lymphocytes, epithelial cells, endothelial cells, muscle cells, fibroblasts (skin cells, etc.), hair cells, hepatocytes, gastric mucosa cells, enterocytes, splenocytes, pancreatic cells (pancreatic exocrine cells, etc.), brain cells, lung cells, kidney cells, adipocytes, and the like, and the like.

The mammal from which the somatic cells are recovered is not particularly limited, and is preferably human.

The proportion of neural crest cells in a cell population containing neural crest cells may vary greatly depending on the collected tissue and differentiation induction conditions. In one embodiment of the purification method 1 of the present invention, the proportion of neural crest cells in a cell population containing neural crest cells may be, but is not limited to, for example, 1 - 95%, 1 - 90%, 1 - 85%, 1 - 80%, 1 - 75%, 1 - 70%, 1 - 65%, 1 - 60%, 1 - 55%, 1 - 50%, 1 - 45%, 1 - 40%, 1 - 35%, 1 - 30%, 1 - 25%, 1 - 20%, 1 - 15%, or 1 - 10%. In another embodiment, the proportion of neural crest cells in a cell population containing neural crest cells may be, but is not limited to, for example, 25 - 95%, 25 - 90%, 25 - 85%, 25 - 80%, 25 - 75%, 25 - 70%, 25 - 65%, 25 - 60%, 25 - 55%, 25 - 50%, 25 - 45%, 25 - 40%, or 25 - 35%. In still another embodiment, the proportion of neural crest cells in a cell population containing neural crest cells may be, but is not limited to, for example, 50 - 95%, 50 - 90%, 50 - 85%, 50 - 80%, 50 - 75%, 50 - 70%, 50 - 65%, or 50 - 60%. In another embodiment, the proportion of neural crest cells in a cell population containing neural crest cells may be, but is not limited to, for example, 75 - 95%, 75 - 90%, or 75 - 85%.

As used herein, the "purification" in the purification method 1 of the present invention means that the cell population containing neural crest cells prepared in step 1 is subjected to step 2 and the proportion of the neural crest cells in the cell population increases exceeding that at the time when the cells were prepared in step 1. In one embodiment, the proportion of the neural crest cells in the cell population after purification by the purification method 1 of the present invention is, but not limited to, 90 - 100%, 91 - 100%, 92 - 100%, 93 - 100%, 94 - 100%, 95 - 100%, 96 - 100%, 97 - 100%, 98 - 100%, 99 - 100%, or 100%.

The cell population containing neural crest cells prepared by the purification method 1 of the present invention may be in the state of single cells by subjecting to, where necessary, dynamic dispersion treatment, dispersing treatment by enzymes such as collagenase, trypsin and the like, and/or dispersing treatment by chelating agents such as EDTA and the like, and the like. When the cell population is processed to make single cells, a ROCK inhibitor may be added to suppress cell death. The ROCK inhibitor is not particularly limited as long as it can suppress the function of Rho-kinase (ROCK). For example, Y-27632, Fasudil/HA1077, H-1152, Wf-536, and derivatives thereof and the like can be mentioned. As the ROCK inhibitor, other known low-molecular-weight compounds can also be used (e.g., US-A-2005/0209261, US-A-2005/0192304, US-A-2004/0014755, US-A-2004/0002508, US-A-2004/0002507, US-A-2003/0125344, US-A-2003/0087919, and WO2003/062227, WO2003/059913, WO2003/062225, WO2002/076976, WO2004/039796).

The cell population prepared in step 1 may be subjected to step 2 after being subjected to a sorting treatment of neural crest cells. Preferably, it is subjected to step 2 without being subjected to a sorting treatment of neural crest cells. Examples of the sorting treatment of neural crest cells include, but are not limited to, sorting treatments such as sorting by a cell sorter using a fluorescence-labeled CD271 specific antibody, sorting by magnetic beads bound with a CD271 specific antibody, sorting using an affinity column with a CD271 specific antibody immobilized thereon, and the like.

In step 2 of the purification method 1 of the present invention, the cell population obtained in step 1 is subjected to expansion culture. In the present specification, the "expansion culture" is a concept including culturing for maintaining and/or proliferating the desired cells, and it may preferably be culturing for proliferation of the desired cells.

The purification method 1 of the present invention aims at purification of neural crest cells. Thus, the culture conditions that may be used are those suitable for maintaining and/or proliferating neural crest cells.

The culture conditions for expansion culturing neural crest cells are not particularly limited as long as they permit expansion culture of the neural crest cells, and culture conditions known per se can be used. One example is a method of culturing in a culture medium containing a TGFβ inhibitor, EGF (epidermal growth factor) and FGF2 (fibroblast growth factor 2).

A medium to be used for expansion culture of neural crest cells can be prepared using a medium used for culturing animal cells as the basal medium. Examples of the basal medium include IMDM medium, Medium199 medium, Eagle's Minimum Essential Medium (EMEM), αMEM medium, Dulbecco's modified Eagle's Medium (DMEM), Ham's F12 medium, RPMI 1640 medium, Fischer's medium, StemPro34 (invitrogen), RPMI-base medium, StemFit (registered trade mark) AK03N medium, and mixed medium of these, and the like. In this step, StemFit (registered trade mark) AK03N medium is preferably used. The medium may contain a serum, or may be serum-free. Where necessary, the medium may contain one or more serum replacements such as albumin, transferrin, Knockout Serum Replacement (KSR) (serum replacement for FBS during ES cell culture), N2 supplement (Invitrogen), B27 supplement (Invitrogen), fatty acid, insulin, collagen precursor, trace element, 2-mercaptoethanol (2ME), thiolglycerol and the like, and may also contain one or more substances such as lipid, amino acid, L-glutamine, Glutamax (Invitrogen), non-essential amino acid, vitamin, growth factor, low-molecular-weight compound, antibiotic, antioxidant, pyruvic acid, buffering agent, inorganic salts and the like.

In the present invention, the TGFβ inhibitor is a substance that inhibits signal transduction from the binding of TGFβ to a receptor and then to SMAD, and is not particularly limited as long as it is a substance that inhibits the binding to a receptor ALK family or a substance that inhibits phosphorylation of SMAD by the ALK family. In the present invention, the TGFβ inhibitor is exemplified by Lefty-1 (e.g., mouse: NM_010094, human: NM_020997 in NCBI Accession No.), SB431542, SB202190 (both R.K. Lindemann et al., Mol. Cancer, 2003, 2:20), SB505124 (GlaxoSmithKline), NPC30345, SD093, SD908, SD208 (Scios), LY2109761, LY364947, LY580276 (Lilly Research Laboratories), A-83-01(WO2009/146408) and derivatives thereof and the like. The TGFβ inhibitor to be used for expansion culture of neural crest cells may be preferably SB431542.

The concentration of a TGFβ inhibitor such as SB431542 and the like in a culture medium is not particularly limited as long as it inhibits ALK5. It is preferably 1 nM - 50 µM (e.g., 1 nM, 10 nM, 50 nM, 100 nM, 500 nM, 750 nM, 1 µM, 2 µM, 3 µM, 4 µM, 5 µM, 6 µM, 7 µM, 8 µM, 9 µM, 10 µM, 15 µM, 20 µM, 25 µM, 30 µM, 40 µM, 50 µM), more preferably 10 µM, though the concentration is not limited to these.

The concentration of EGF in a medium is preferably 1 ng/ml - 100 ng/ml (e.g., 1 ng/ml, 5 ng/ml, 10 ng/ml, 20 ng/ml, 30 ng/ml, 40 ng/ml, 50 ng/ml, 60 ng/ml, 70 ng/ml, 80 ng/ml, 90 ng/ml, 100 ng/ml), more preferably 20 ng/ml, though the concentration is not limited to these.

The concentration of FGF2 in a medium is preferably 1 ng/ml - 100 ng/ml (e.g., 1 ng/ml, 5 ng/ml, 10 ng/ml, 20 ng/ml, 30 ng/ml, 40 ng/ml, 50 ng/ml, 60 ng/ml, 70 ng/ml, 80 ng/ml, 90 ng/ml, 100 ng/ml), more preferably 20 ng/ml, though the concentration is not limited to these.

A component other than the above-mentioned components can also be added to the medium as long as the expansion culture of neural crest cells can be achieved.

In step 2, the expansion culture of neural crest cells may be performed by any of adhesion culture and suspension culture. It is preferably performed by adhesion culture.

The purification method 1 of the present invention is characterized by the use of laminin 211 to achieve purification of neural crest cells.

Laminin is a glycoprotein that is the main constituent molecule of the basement membrane. Laminin is known to be involved in various cell functions such as cell adhesion, cell proliferation, metastasis, differentiation and the like. Laminin is composed of a heterotrimer having one each of α, β, and γ subunit chains. Currently, 5 types of α subunit chains (α1, α2, α3, α4, α5), 3 types of β subunit chains (β1, β2, β3), and 3 types of γ subunit chains (γ1, γ2, γ3) are known to exist. Depending on the combination of these subunit chains, there are 15 types of laminin isoforms currently confirmed to be present in humans. Laminin 211 to be used in the purification method of the present invention is a laminin composed of subunit chains of α2 chain, β1 chain, and γ1 chain. The origin of laminin is preferably the same as the organism from which the neural crest cells are derived (for example, when human-derived neural crest cells are used, it is preferable to use human-derived laminin 211). Laminin is known to have stronger cell adhesion activity in the laminin E8 fragment, which is composed only of the integrin binding site, than in full-length laminin (Miyazaki T. et al., Nat Commun. 2012; 3:1236). Laminin 211 to be used in the purification method of the present invention is not a fragment but a laminin 211 full-length protein. Laminin 211 may be prepared using gene recombination technology known per se, or commercially available one may be used.

In step 2, when the cell population is expansion cultured during suspension culture, laminin 211 may be added to the medium so that the suspending cells can use laminin 211 as a scaffold. The suspension culture can be performed by a method known per se. One example is a method of expansion culturing a cell population during suspension culture in a medium containing laminin 211 while stirring the medium using a spinner flask or the like. Alternatively, a cell population can also be expansion cultured during suspension culture by using laminin 211 in combination with a polysaccharide (e.g., methyl cellulose, xanthan gum, gellan gum, etc.) affording the effect of suspending cells when added to the medium. The concentration, and the like of laminin to be added may be appropriately set in consideration of various conditions such as seeding density of cell population, concentration of polysaccharide to be used in combination, and the like. In the present specification, the suspension culture means a culture method in which cells are cultured without adhesion of the cells to the surface of a culture container. Suspension culture may or may not accompany physical agitation. In addition, the cells to be cultured may be uniformly dispersed or non-uniformly dispersed in the medium.

In step 2, when a cell population is expansion cultured in adhesion culture, laminin 211 is coated on the surface of a culture container. The coating amount of laminin 211 is not particularly limited as long as the desired effect of the present invention can be obtained, and a generally recommended coating amount may be used. As one example, the coating amount of laminin 211 is 0.1 ng/cm² - 1000 ng/cm², preferably 0.5 ng/cm² - 500 ng/cm², more preferably 1 ng/cm² - 250 ng/cm², further preferably 2 ng/cm² - 100 ng/cm², though not limited to these.

The culture period in step 2 varies depending on the culture conditions, culture method, proportion of neural crest cells in the cell population, and the like, and the neural crest cells can be purified in a relatively short period of time. One example of the culture period is 1 - 21 days, 1 - 20 days, 1 - 19 days, 1 - 18 days, 1 - 17 days, 1 - 16 days, 1 - 15 days, 1 - 14 days, 1 - 13 days, 1 - 12 days, 1 - 11 days, 1 - 10 days, 1 - 9 days, 1 - 8 days, 1 - 7 days, 1 - 6 days, 1 - 5 days, 1 - 4 days, or 1 - 3 days, though not limited to these.

The culture temperature in step 2 is not particularly limited as long as the neural crest cells can be cultured, and is 30 - 40°C, preferably about 37°C. The CO₂ concentration during culture in step 2 is not particularly limited as long as the neural crest cells can be cultured, and is 2 - 5%, preferably about 5%.

### 2. Production method of neural crest cells

The present invention also provides a method for producing purified neural crest cells, including the following steps (hereinafter sometimes to be referred to as "production method 1 of the present invention"):
step 1) preparing a cell population containing neural crest cells, and step 2) expansion culturing the cell population obtained in step 1, by using laminin 211 as a scaffold.

Various conditions for the production method of the present invention are the same as those in the purification method 1 of the present invention.

### 3. Purification method of corneal epithelial cells

The present invention provides a method for purifying corneal epithelial cells, including the following steps (hereinafter sometimes to be referred to as "purification method 2 of the present invention"):
step 1) obtaining a cell population comprising corneal epithelial cells in a proportion of not less than 25%, and
step 2) expansion culturing the cell population obtained in step 1, by using laminin 332 as a scaffold.

The "corneal epithelial cell (also referred to as "CEC")" is a cell constituting the outermost corneal epithelial layer of the cornea. The corneal epithelial cell is derived from epidermis ectoderm. The term "corneal epithelial cell" in the present specification includes not only the cells collected from living organisms but also pluripotent stem cell-derived corneal epithelial cells, and cells that have been passaged therefrom. In the purification method 2 of the present invention, the origin of the corneal epithelial cells is not particularly limited, and they may be derived from any vertebrate, and corneal epithelial cells derived from a mammal are preferred. Examples of such mammal include, but are not limited to, mouse, rat, guinea pig, hamster, rabbit, cat, dog, sheep, swine, bovine, horse, goat, monkey, and human.

In addition, the term "corneal epithelial cell" in the present specification is a concept that may include corneal epithelial stem cells and/or corneal epithelial progenitor cells.

As a method for preparing a cell population containing corneal epithelial cells in step 1 of the purification method 2 of the present invention, in the case of a cell population containing corneal epithelial cells derived from living organism, a cell population can be prepared by collecting the cell population from corneal epithelium layer. In the case of a cell population containing pluripotent stem cell-derived corneal epithelial cells, a known preparation method may be adopted, as described above. One example is the method shown in the present Examples, or a method of culturing pluripotent stem cells in a serum-free medium containing no BMP for a certain period of time in the presence of stromal cells or amniotic membrane-derived factors. By these methods, pluripotent stem cells (e.g., iPS cells) are differentiated into SEAM. SEAM may include corneal epithelial cells.

Whether the thus-obtained cell population contains corneal epithelial cells may be known by confirming the expression of one or more corneal epithelial cell-specific marker genes such as PAX-6, Cytokeratin 12, Cytokeratin 3 and the like by a method known per se. It is preferable that the cell population containing corneal epithelial cells used in the purification method 2 of the present invention is derived from pluripotent stem cell, more preferably, iPS cell. The "pluripotent stem cell" in the present invention is as described above.

The proportion of corneal epithelial cells in a cell population containing corneal epithelial cells may vary greatly depending on the collected tissue and differentiation induction conditions. In one embodiment of the purification method 2 of the present invention, the proportion of corneal epithelial cells in a cell population containing corneal epithelial cells may be, but is not limited to, for example, 1 - 95%, 1 - 90%, 1 - 85%, 1 - 80%, 1 - 75%, 1 - 70%, 1 - 65%, 1 - 60%, 1 - 55%, 1 - 50%, 1 - 45%, 1 - 40%, 1 - 35%, 1 - 30%, 1 - 25%, 1 - 20%, 1 - 15%, or 1 - 10%. In another embodiment, the proportion of corneal epithelial cells in a cell population containing corneal epithelial cells may be, but is not limited to, for example, 25 - 95%, 25 - 90%, 25 - 85%, 25 - 80%, 25 - 75%, 25 - 70%, 25 - 65%, 25 - 60%, 25 - 55%, 25 - 50%, 25 - 45%, 25 - 40%, or 25 - 35%. In still another embodiment, the proportion of corneal epithelial cells in a cell population containing corneal epithelial cells may be, but is not limited to, for example, 50 - 95%, 50 - 90%, 50 - 85%, 50 - 80%, 50 - 75%, 50 - 70%, 50 - 65%, or 50 - 60%. In another embodiment, the proportion of corneal epithelial cells in a cell population containing corneal epithelial cells may be, but is not limited to, for example, 75 - 95%, 75 - 90%, or 75 - 85%.

In a cell population containing less than 25% of corneal epithelial cells, the proportion of the corneal epithelial cells can be made not less than 25% by a sorting treatment of the corneal epithelial cells. The sorting treatment of corneal epithelial cells can be performed by a method known per se. One example is, though not limited to, a method including confirming a marker specifically expressed on the surface of corneal epithelial cells and selecting a cell expressing the marker.

In the purification method 2 of the present invention, corneal epithelial cells can be purified by expanding and culturing a cell population containing not less than 25% of corneal epithelial cells by using laminin 332 as a scaffold. The proportion of corneal epithelial cells in a cell population is generally not less than 25%, preferably not less than 30%, not less than 35%, not less than 40%, not less than 45%, not less than 50%, not less than 55%, not less than 60%, not less than 65%, not less than 70%, not less than 75%, not less than 80%, not less than 85%, or not less than 90%, though the proportion is not limited to these.

The "purification" in the purification method 2 of the present invention means that the cell population containing corneal epithelial cells prepared in step 1 is subjected to step 2 and the proportion of the corneal epithelial cells in the cell population increases exceeding that at the time when the cells were prepared in step 1. In one embodiment, the proportion of the corneal epithelial cells in the cell population after purification by the purification method 2 of the present invention is, but not limited to, 90 - 100%, 91 - 100%, 92 - 100%, 93 - 100%, 94 - 100%, 95 - 100%, 96 - 100%, 97 - 100%, 98 - 100%, 99 - 100%, or 100%.

The cell population containing corneal epithelial cells prepared by step 1 in the purification method 2 of the present invention may be in the state of single cells by subjecting to, where necessary, dynamic dispersion treatment, dispersing treatment by enzymes such as collagenase, trypsin and the like, and/or dispersing treatment by chelating agents such as EDTA and the like, and the like. When the cell population is processed to make single cells, a ROCK inhibitor may be added to suppress cell death. The ROCK inhibitor is as described above.

The cell population prepared in step 1 may be subjected to step 2 after being subjected to a sorting treatment of corneal epithelial cells. Preferably, it is subjected to step 2 without being subjected to a sorting treatment of corneal epithelial cells.

In step 2 of the purification method 2 of the present invention, the cell population obtained in step 1 is subjected to expansion culture. The meaning of the "expansion culture" in the present specification is as described above.

The purification method 2 of the present invention aims at purification of corneal epithelial cells. Thus, the culture conditions that may be used are those suitable for maintaining and/or proliferating corneal epithelial cells.

The culture conditions for expansion culturing corneal epithelial cells are not particularly limited as long as they permit expansion culture of the corneal epithelial cells, and culture conditions known per se can be used. One example is a method of culturing in a culture medium containing a KGF (Keratinocyte growth factor) and a Rho kinase inhibitor.

A medium to be used for expansion culture of corneal epithelial cells can be prepared using a medium used for culturing animal cells as the basal medium. Examples of the basal medium include IMDM medium, Medium199 medium, Eagle's Minimum Essential Medium (EMEM), αMEM medium, Dulbecco's modified Eagle's Medium (DMEM), Ham's F12 medium, RPMI 1640 medium, Fischer's medium, StemPro34 (invitrogen), RPMI-base medium, StemFit (registered trade mark) AK03N medium, and mixed medium of these, and the like. In this step, StemFit (registered trade mark) AK03N medium is preferably used. The medium may contain a serum, or may be serum-free. Where necessary, the medium may contain one or more serum replacements such as albumin, transferrin, Knockout Serum Replacement (KSR) (serum replacement for FBS during ES cell culture), N2 supplement (Invitrogen), B27 supplement (Invitrogen), fatty acid, insulin, collagen precursor, trace element, 2-mercaptoethanol (2ME), thiolglycerol and the like, and may also contain one or more substances such as lipid, amino acid, L-glutamine, Glutamax (Invitrogen), non-essential amino acid, vitamin, growth factor, low-molecular-weight compound, antibiotic, antioxidant, pyruvic acid, buffering agent, inorganic salts and the like.

The concentration of KGF in the culture medium is not particularly limited as long as it permits proliferation of corneal epithelial cells. It is preferably 0.1 - 200 ng/mL (e.g., 0.1 ng/mL, 1 ng/mL, 10 ng/mL, 20 ng/mL, 30 ng/mL, 40 ng/mL, 50 ng/mL, 60 ng/mL, 70 ng/mL, 80 ng/mL, 90 ng/mL, 100 ng/mL, 110 ng/mL, 120 ng/mL, 130 ng/mL, 140 ng/mL, 150 ng/mL, 160 ng/mL, 170 ng/mL, 180 ng/mL, 190 ng/mL, 200 ng/mL), though the concentration is not limited to these. The concentration is more preferably 20 ng/ml.

A component other than the above-mentioned components can also be added to the medium as long as the expansion culture of corneal epithelial cells can be achieved.

In step 2, the expansion culture of corneal epithelial cells may be performed by any of adhesion culture and suspension culture. It is preferably performed by adhesion culture.

The purification method 2 of the present invention is characterized by the use of laminin 332 as a scaffold to achieve purification of corneal epithelial cells.

Laminin 332 to be used in the purification method 2 of the present invention is not a fragment but a laminin 332 full-length protein. Laminin 332 may be prepared using gene recombination technology known per se, or commercially available one may be used.

In step 2, when the cell population is expansion cultured during suspension culture, laminin 332 may be added to the medium so that the suspending cells can use laminin 332 as a scaffold. The method and conditions for suspension culture are as described above.

In step 2, when a cell population is expansion cultured in adhesion culture, laminin 332 is coated on the surface of a culture container. The coating amount of laminin 332 is not particularly limited as long as the desired effect of the present invention can be obtained, and a generally recommended coating amount may be used. As one example, the coating amount of laminin 332 is more than 0.01 µg/cm² and less than 0.5 µg/cm², preferably more than 0.05 µg/cm² and less than 0.4 µg/cm², more preferably more than 0.05 µg/cm² and less than 0.3 µg/cm², further preferably not less than 0.05 µg/cm² and less than 0.25 µg/cm², though not limited to these.

The culture period in step 2 varies depending on the culture conditions, culture method, proportion of corneal epithelial cells in the cell population, and the like, and can be appropriately set. One example of the culture period is 1 - 50 days, 1 - 40 days, 1 - 30 days, 1 - 20 days, 1 - 17 days, 1 - 16 days, 1 - 15 days, 1 - 14 days, 1 - 13 days, 1 - 12 days, 1 - 11 days, 1 - 10 days, 1 - 9 days, 1 - 8 days, 1 - 7 days, 1 - 6 days, 1 - 5 days, 1 - 4 days, or 1 - 3 days, though not limited to these.

The culture temperature in step 2 is not particularly limited as long as the corneal epithelial cells can be cultured, and is 30 - 40°C, preferably about 37°C. The CO₂ concentration during culture in step 2 is not particularly limited as long as the corneal epithelial cells can be cultured, and is 2 - 5%, preferably about 5%.

### 4. Production method of corneal epithelial cells

The present invention also provides a method for producing a purified corneal epithelial cell, including the following steps (hereinafter sometimes to be referred to as "production method 2 of the present invention"):
step 1) preparing a cell population containing corneal epithelial cells, and step 2) expansion culturing the cell population obtained in step 1, by using laminin 332 as a scaffold.

Various conditions for the production method 2 of the present invention are the same as those for the purification method 2 of the present invention.

The present invention is explained further specifically in the following Examples; however, the present invention is not limited by these examples.

### [Example]

### [Experimental Example 1] Evaluation of scaffold function of laminin 211 in iPS cell culture (material and method)

The iPS cell line used was 201B7 (iPS portal). The cells were seeded on a 24-well plate coated with laminin 511-E8 fragment (iMatrix511, Nippi: 0.26 - 6.58 µg/cm²), or laminin 211 (Biolamina: 0.05 - 6.58 µg/cm²) at 2300 cells/well, cultured in StemFit^{®} AK03N (Ajinomoto Co., Inc.) medium at 37°C under 5% CO₂ for 5 days, and cell adhesion and colony formation were evaluated. Cell adhesion was evaluated by observation under a microscope on day 5 of culture. Cell proliferation was evaluated by determining colony formation by observation under a microscope on day 0 and day 5 of culture, and comparing the colony formation. The results are shown in Table 1.

**[Table 1]**

| scaffold material | amount of coating (µg/cm²) | cell adhesion | colony formation |
|---|---|---|---|
| **Laminin 211** (recommended amount of coating: 0.5 - 2 µg/cm²) | 0.05 | × | × |
| | 0.26 | × | × |
| | 1.32 | × | × |
| | 6.58 | × | × |
| **Laminin 511-E8 fragment** (recommended amount of coating: 0.5 - 2 µg/cm²) | 0.26 | ○ | ○ |
| | 1.32 | ○ | ○ |
| | 6.58 | ○ | ○ |
| **Non-coat** | 0 | × | × |

As shown in Table 1, when laminin 511-E8 fragment was used as a scaffold, cell adhesion and colony formation were good irrespective of the amount of coating. On the other hand, when laminin 211 was used as a scaffold, neither cell adhesion nor colony formation was observed irrespective of the amount of coating, as in the negative target (non-coat).

### [Example 1] Production of neural crest cells from iPS cells (material and method)

### 1. Induction of differentiation of iPS cells into neural crest cells

The iPS cell line used was 201B7 (iPS portal). The cells were seeded on a 6-well plate coated with laminin 511-E8 fragment (iMatrix511, Nippi) at 6500 cells/well, cultured in StemFit^{®} AK03N (Ajinomoto Co., Inc.) medium at 37°C under 5% CO₂ for 5 days. Then, differentiation into neural crest cells was induced at 37°C under 5% CO₂ for 14 days in a medium obtained by adding SB431542 (Stemgent, Inc., 10 µM) and CHIR99021 (Wako, 0.3 µM (conditions 1) or 0.9 µM (conditions 2) to StemFit AK03N (Solution A+Solution B). The proportion of neural crest cells on completion of the induction was calculated by determining the proportion of CD271 protein-high expressing cells by using FACS. In addition, the gene expression of neural crest cell markers was analyzed by RT-PCR. The primers used in the RT-PCR method are shown below.

marker gene Primer ID Taqman Cat.No.
TFAP2a Hs00271528_CE A15629
SOX9 Hs01001343_g1 4331182
TWIST1 Hs01675818 s1 4331182
(β actin was used as reference gene (Hs01101944_s1, 4331182))

### 2. Selective proliferation of neural crest cells

The cell population containing neural crest cells (SEAM) which was prepared the above-mentioned 1. was single-celled using TrypLE Select (Thermo Fisher). The single-celled cell population was seeded on 6-well plates coated with various scaffold materials, and cultured under culture conditions suitable for the expansion culture of neural crest cells. More specifically, the cells were cultured at 37°C under 5% CO₂ for 9 - 10 days in a medium obtained by adding SB431542 (10 µM), Epithelium growth factor (Sigma Chemical, 20 ng/mL) and StemFit AK03N Solution C (Ajinomoto Co., Inc., 0.08%) to StemFit AK03N (Solution A+Solution B). After 9 - 10 days of culture, when the confluence reached about 90%, the proportion of the neural crest cells in the cell population cultured with each scaffold material and the expression state of neural crest cell gene markers were determined by methods similar to those in the above-mentioned 1.

Scaffolding materials used were as follows.
(1) laminin 511-E8 fragment (Nippi, 31.3 ng/cm² and 312.5 ng/cm²)
(2) laminin 211 (Biolamina, 6.3 ng/cm² and 62.5 ng/cm²)
(3) Vitronectin (Life Technologies, 31.3 ng/cm² and 312.5 ng/cm²)
(4) Fibronectin (Sigma Chemical, 1562.5 ng/cm² and 3125.0 ng/cm²)

Each of the scaffold materials (1) - (3) was applied to the surface of a 6-well plate by directly suspending the materials in the culture medium. The scaffold material of (4) was applied to the surface of a 6-well plate by dissolving the material in 1 mL of PBS(-), and adding same to the well, followed by allowing to stand at room temperature for 1 hr.

The results are shown in Tables 2 and 3.

**[Table 2]**

| differentiation induction conditions | proportion of NCC at the end of induction | cell population containing NCC after induction was expansion cultured with each scaffold (conditions 1: 10 days, conditions 2: 9 days) | | | | | | | | |
|---|---|---|---|---|---|---|---|---|---|---|
| | | Laminin 511-E8 fragment | | Laminin 211 | | Vitronectin | | Fibronectin | | Non-coat |
| | | amount of coating | proportion of NCC | amount of coating | proportion of NCC | amount of coating | proportion of NCC | amount of coating | proportion of NCC | proportion of NCC |
| conditions 1 CHIR 0.3 µM | C | 31.3 | B | 6.3 | A | 31.3 | B | 1562.5 | E | B |
| conditions 2 CHIR 0.9 µM | A | 31.3 | B | 6.3 | A | 31.3 | B | 1562.5 | B | B |
| | | 312.5 | D | 62.5 | A | 312.5 | C | 3125 | D | |

| | | | | | | | | | | |
|---|---|---|---|---|---|---|---|---|---|---|
| **(proportion of NCC) E: ≤50%, D:51 - 75%, C:76 - 85%, B:86 - 95%, A:>95%** | | | | | | | | | | |

**[Table 3]**

| | | | | | | | | | | |
|---|---|---|---|---|---|---|---|---|---|---|
| induction conditions 1 | | | | | | | | | | |

| marker | at the end of induction | Laminin 511-E8 fragment (31.3 ng/cm²) | Laminin 211 (6.3 ng/cm²) | Vitronectin (31.3 ng/cm²) | Fibronectin (1562.5 ng/cm²) | Non-coat | Laminin 511-E8 fragment (312.5 ng/cm²) | Laminin 211 (62.5 ng/cm²) | Vitronectin (312.5 ng/cm²) | Fibronectin (3125 ng/cm²) |
|---|---|---|---|---|---|---|---|---|---|---|
| TFAP2a | B | A | A | A | A | A | | | | |
| SOX9 | C | A | A | A | A | A | | | | |
| TWIST1 | A | AAA | AA | AA | AA | AA | | | | |
| induction conditions 2 | | | | | | | | | | |
| TFAP2a | A | A | A | A | A | A | A | A | A | A |
| SOX9 | B | A | A | A | A | A | A | A | A | A |
| TWIST1 | A | AAA | AA | AAA | AA | AAA | AAA | AAA | AAA | AA |

| | | | | | | | | | | |
|---|---|---|---|---|---|---|---|---|---|---|
| **C:<1, 1≤B<5, 5≤A<50, 50≤AA<500, 500≤AAA (expression level of β actin as 1)** | | | | | | | | | | |

As shown in Table 2, the proportion of neural crest cells in a cell population containing neural crest cells differentiated from iPS cells could be increased remarkably by expansion culture of the cell population by using laminin 211 as a scaffold, irrespective of the proportion of the neural crest cells in the cell population subjected to the expansion culture or the amount of laminin 211 coated on the surface of the culture container.

### [Example 2] Cell proliferation test

The purified neural crest cells obtained by expansion culture (9 days) of a cell population under differentiation induction conditions 2 by using each scaffold material in Example 1 were recovered, re-seeded on a 6-well plate coated with each scaffold material, and further cultured for 5 days under the culture conditions used in the purification step of Example 1. The cells were single-celled by using TrypLE Select after 5 days of culture, and counted. The results are shown in Table 4.

**[Table 4]**

| purified neural crest cells obtained by 9 days of expansion culture of SEAM were further expansion cultured for 5 days on each scaffold material | | | | | | | | |
|---|---|---|---|---|---|---|---|---|
| Laminin 511-E8 fragment | | Laminin 211 | | Vitronectin | | Fibronectin | | Non-coat |
| amount of coating | cell proliferation rate in two passages | amount of coating | cell proliferation rate in two passages | amount of coating | cell proliferation rate in two passages | amount of coating | cell proliferation rate in two passages | cell proliferation rate in two passages |
| 31.3 | C | 6.3 | B | 31.3 | A | 1562.5 | B | C |
| 312.5 | C | 62.5 | B | 312.5 | C | 3125 | D | |

| | | | | | | | | |
|---|---|---|---|---|---|---|---|---|
| **(proliferation rate) E: <10-fold, D: 10- to 99-fold, C: 100- to 499-fold, B: 500- to 999-fold, A: ≥1000-fold** | | | | | | | | |

As shown in Table 4, the cell proliferation activity of neural crest cells purified using laminin 211 as a scaffold was fine. Considering the very high purity of the neural crest cells purified using laminin 211 as a scaffold as shown in the above-mentioned Table 2, it is suggested that expansion culture using laminin 211 is extremely preferable for the production of neural crest cells having high purity.

### [Experimental Example 2] Evaluation of scaffold function of laminin 332 in iPS cell culture (material and method)

iPS cell line 201B7 (iPS portal) was seeded on a 12-well plate coated with Laminin 511-E8 fragment (iMatrix511, Nippi: 0.05 - 5.00 µg/cm²) or Laminin 332 (Biolamina: 0.05 - 5.00 µg/cm²) at 2500 cells/well and cultured for 5 days, and the cell adhesion and proliferation were evaluated. The results of cell adhesion on day 5 of culture from the next day of seeding and cell proliferation on day 5 of culture from the next day of seeding are shown in Table 5.

**[Table 5]**

| scaffold material | amount of coating (µg/cm²) | cell adhesion | colony formation |
|---|---|---|---|
| **Laminin 332** (recommended amount of coating: 0.5 - 2 µg/cm²) | 0.05 | × | × |
| | 0.25 | × | × |
| | 0.50 | ○ | ○ |
| | 1.00 | ○ | ○ |
| | 5.00 | ○ | ○ |
| **Laminin 511-E8 fragment** (recommended amount of coating: 0.5 - 2 µg/cm²) | 0.05 | ○ | ○ |
| | 0.25 | ○ | ○ |
| | 0.50 | ○ | ○ |
| | 1.00 | ○ | ○ |
| | 5.00 | ○ | ○ |
| **Non-coat** | 0.00 | × | × |

As shown in Table 5, with the Laminin 511-E8 fragment, iPS cells adhered and proliferated at any amount of coating. On the other hand, with the Laminin 332, iPS cells adhered and proliferated at a coating amount of more than 0.50 µg/cm².

### [Example 3] Production of corneal epithelial cells from iPS cells (material and method)

### 1. Induction of corneal epithelial cells from iPS cells

The iPS cell line used was 201B7 (iPS portal). The iPS cells were seeded on a 12-well plate coated with Laminin 511-E8 fragment (iMatrix511, Nippi: 0.25, 0.50, or 5.00 µg/cm²) or Laminin 332 (Biolamina: 0.5 or 5.00 µg/cm²) at 2500 cells/well, cultured in StemFit^{®} AK03N medium at 37°C under 5% CO₂ for 5 days. Then, the medium was exchanged with a medium obtained by adding SB431542 (Stemgent, Inc., 10 µM) and CHIR99021 (Wako, 0.6 µM) to StemFit AK03N (Solution A+Solution B) and differentiation into corneal epithelial cells was induced at 37°C under 5% CO₂ for 15 days. The differentiation induction rate of the corneal epithelial cells was evaluated by determining the proportions of corneal epithelial cell marker PAX-6 and Cytokeratin12 protein-expressing cells by using FACS. The proportion of the corneal epithelial cells obtained on completion of the induction is shown in Table 6.

**[Table 6]**

| scaffold material for induction | | proportion (%) of PAX-6 and Cytokeratin12 positive cells at the end of induction |
|---|---|---|
| | (µg/cm²) | |
| Laminin 511-E8 | 0.25 | A |
| | 0.50 | B |
| | 5.00 | D |
| Laminin 332 | 0.50 | D |
| | 5.00 | B |

| | | |
|---|---|---|
| **(proportion of CEC) D: <25%, C: 25 - 49%, B: 50 - 74%, A: 7589%, AA: ≥90%** | | |

### 2. Selective proliferation of corneal epithelial cells

The cell population containing corneal epithelial cells (SEAM) which was prepared the above-mentioned 1. was single-celled using TrypLE Select (Thermo Fisher). The single-celled cell population was seeded on a 6-well plate coated with Laminin 332 at a concentration of 0.06 µg/cm². at 2×10⁵ cells/well, and cultured under culture conditions suitable for the expansion culture of corneal epithelial cells. More specifically, the cells were cultured at 37°C under 5% CO₂ in a medium obtained by adding Keratinocyte growth factor (Peprotech, 20 ng/mL) and Y-27632 (Wako, 10 µM) to StemFit AK03N (Solution A+Solution B). The proportion of the corneal epithelial cells during purifying culture 1 passage was determined, as in the aforementioned 1., by evaluating the proportions of corneal epithelial cell marker PAX-6 and Cytokeratin12 protein-expressing cells by using FACS. The results are shown in Table 7.

**[Table 7]**

| scaffold material for induction | | cell population containing corneal epithelial cells obtained under each induction condition was expansion cultured (1 passage) using laminin 332 (amount of coating: 0.06 µg/cm²) as scaffold | |
|---|---|---|---|
| | (µg/cm²) | proportion (%) of PAX-6 and Cytokeratin12 positive cells | cell increase rate |
| Laminin 511-E8 | 0.25 | AA | 6.0 |
| | 0.50 | A | 3.1 |
| | 5.00 | (cells grew only insufficiently) | 0.2 |
| Laminin 332 | 0.50 | (cell grew only insufficiently) | 1.1 |
| | 5.00 | AA | 3.8 |

| | | | |
|---|---|---|---|
| **(proportion of CEC) C: <50%, B: 50 - 74%, A: 75 - 89%, AA: ≥90%** | | | |

At the time of purifying culture 1 passage, the cells did not grow sufficiently under conditions where the proportion of PAX-6 and Cytokeratin12 positive cells (i.e., CEC) is less than 25% at the end of induction. However, the proportion of PAX-6 and Cytokeratin12 positive cells was improved under any conditions where the proportion of PAX-6 and Cytokeratin12 positive cells was not less than 25% at the end of induction.

### [Industrial Applicability]

According to the present invention, neural crest cells can be purified conveniently in a very short period of time from a cell population containing neural crest cells. According to the present invention, moreover, high-purity neural crest cells can be prepared conveniently in a very short period of time. Furthermore, according to the present invention, corneal epithelial cells can be purified conveniently from a cell population containing corneal epithelial cells. According to the present invention, moreover, high-purity corneal epithelial cells can be produced conveniently. Therefore, the present invention is extremely useful in the field of, for example, regenerative medicine.

This application is based on a patent application No. 2019-091988 filed in Japan (filing date: May 15, 2019) and a patent application No. 2019-186280 filed in Japan (filing date: October 9, 2019), the contents of which are incorporated in full herein.

## Claims

1. A method for purifying a neural crest cell, comprising the following steps:
step 1) obtaining a cell population comprising neural crest cells, and
step 2) expansion culturing the cell population obtained in step 1, by using laminin 211 as a scaffold.

2. The method according to claim 1, wherein the cell population comprising neural crest cells obtained in step 1 is subjected to step 2 without being subjected to a sorting treatment of the neural crest cells.

3. The method according to claim 1 or 2, wherein the culture period in step 2 is 1 - 21 days.

4. The method according to any one of claims 1 to 3, wherein the cell population comprising neural crest cells is derived from a pluripotent stem cell.

5. The method according to claim 4, wherein the pluripotent stem cell is an iPS cell.

6. A method for producing a purified neural crest cell, comprising the following steps:
step 1) obtaining a cell population comprising neural crest cells, and
step 2) expansion culturing the cell population obtained in step 1, by using laminin 211 as a scaffold.

7. The method according to claim 6, wherein the cell population comprising neural crest cells obtained in step 1 is subjected to step 2 without being subjected to a sorting treatment of the neural crest cells.

8. The method according to claim 6 or 7, wherein the culture period in step 2 is 1 - 21 days.

9. The method according to any one of claims 6 to 8, wherein the cell population comprising neural crest cells is derived from a pluripotent stem cell.

10. The method according to claim 9, wherein the pluripotent stem cell is an iPS cell.

11. A method for purifying a corneal epithelial cell, comprising the following steps:
step 1) obtaining a cell population comprising corneal epithelial cells in a proportion of not less than 25%, and
step 2) expansion culturing the cell population obtained in step 1, by using laminin 332 as a scaffold.

12. The method according to claim 11, wherein the cell population comprising corneal epithelial cells obtained in step 1 is subjected to step 2 without being subjected to a sorting treatment of the corneal epithelial cells.

13. The method according to claim 11 or 12, wherein the laminin 332 used as the scaffold in the expansion culture in step 2 is coated in an amount of more than 0.01 µg/cm². and less than 0.5 µg/cm².

14. The method according to any one of claims 11 to 13, wherein the cell population comprising corneal epithelial cells is derived from a pluripotent stem cell.

15. The method according to claim 14, wherein the pluripotent stem cell is an iPS cell.

16. A method for producing a purified corneal epithelial cell, comprising the following steps:
step 1) obtaining a cell population comprising corneal epithelial cells in a proportion of not less than 25%, and
step 2) expansion culturing the cell population obtained in step 1, by using laminin 332 as a scaffold.

17. The method according to claim 16, wherein the cell population comprising corneal epithelial cells obtained in step 1 is subjected to step 2 without being subjected to a sorting treatment of the corneal epithelial cells.

18. The method according to claim 16 or 17, wherein the laminin 332 used as the scaffold in the expansion culture in step 2 is coated in an amount of more than 0.01 µg/cm² and less than 0.5 µg/cm².

19. The method according to any one of claims 16 to 18, wherein the cell population comprising corneal epithelial cells is derived from a pluripotent stem cell.

20. The method according to claim 19, wherein the pluripotent stem cell is an iPS cell.
